# EUROPEAN PATENT APPLICATION

(11) **EP 2 548 555 A1**
(43) Date of publication of application: **23.01.2013**
(21) Application number: 11174323.3
(22) Date of filing: 18.07.2011
(51) Int. Cl.: A61K 31/4439

(54) **Proton pump inhibitors as immunomodulators**

(71) Applicant: Fondazione IRCCS Istituto Nazionale dei Tumori, 20133 Milan (IT); Ospedale San Raffaele S.r.l., 20132 Milano (IT); Istituto Superiore di Sanità, 00161 Roma (IT)
(72) Inventor: Rivoltini, Licia, 20133 Milano (IT); Bellone, Matteo, 20132 Milano (IT); Fais, Stefano, 00136 Roma (IT)
(74) Representative: Zaccaro, Elisabetta

(57) **Abstract**

The present invention relates to Proton Pump Inhibitors (PPI) for use as immunomodulators which allow to overcome immune escape in antineoplastic therapy, chronic inflammatory diseases associated with an acidic microenvironment and in chronic infection thereto associated.

The therapeutic efficacy of PPIs is guaranteed by their ability of inhibiting the activity of the hydrogen/potassium adenosine triphosphatase enzyme system (H⁺/K⁺- ATPase or proton pump).

The PPI according to the present invention is advantageously selected from the group consisting of omeprazole, lansoprazole, pantoprazole, esomeprazole, dexlansoprazole, rabeprazole, tenatoprazole or a mixture thereof.

Additionally, the disclosure describes a vaccine composition comprising at least one Proton Pump Inhibitor and pharmaceutically acceptable excipients.

## Description

### Field of the invention

The present invention relates to Proton Pump Inhibitors for use as immunomodulators. Additionally, the disclosure describes a vaccine composition comprising at least one Proton Pump Inhibitor and pharmaceutically acceptable excipients.

### State of the art

Several lines of evidence suggest that T cell immunity may play a role in controlling tumor development. Tumor-infiltrating T lymphocytes (TILs) with an effector/memory phenotype are associated with a more favorable prognosis in cancer patients and mediate tumor regression when adoptively transferred after ex-vivo activation.

However, TILs are reported to acquire functional defects at the tumor site and enter a state of reversible anergy that is mostly attributed to suboptimal TCR ligation, a lack of TCR and CD8 co-localization, or inhibitory signaling through CTLA4. This may, at least in part, explain the clinical observation that TILs can be abundantly detected in progressing cancer lesions (Parmiani G, Castelli C, Santinami M, Rivoltini L.; Curr Opin Oncol 2007;19(2):121-7).

An issue frequently neglected relates to the metabolic features of the tumor microenvironment, which represents a potentially hostile milieu due to the hypoxic and acidic conditions that occur as a consequence of inadequate blood flow, inflammation, enhanced glycolysis (the so-called "Warburg effect") and the production of acidic metabolites (Gatenby RA, Gillies RJ.; Nat Rev Cancer 2004; 4(11):891-9; Fais S, De Milito A, You H, Qin W.; Cancer Res. 2007 Nov 15;67(22):10627-30.).

In addition, excessive CO₂ release caused by altered glycolysis leads to increased tumor expression of carbonic anhydrase IX (CA-IX), thus contributing to further acidification of the extracellular tumor environment. Hypoxia also activates the HIF pathway, which in turn upregulates glucose transporters and CA-IX and leads to additional exacerbation of tumor acidosis. As a result, the extracellular pH can drop to values of 6.0 or less and is on average 0.2-1.0 units lower than that of normal tissues.

This biochemical shift has disadvantageously been shown to provide a selective growth advantage to tumor cells at the expense of infiltrating host cells, including immune cells. While the impact of these metabolic alterations on TILs is presently unknown, the clinical evidence that metabolic acidosis is often associated with immunodeficiency (Kellum JA.; Crit Care Resusc 2004;6(3):197-203), and that both leukocyte activation and the bactericidal capacity of leukocytes are generally impaired at reduced pH (Lardner A.; J Leukoc Biol 2001;69(4):522-30), suggests that T cells could be extremely sensitive to pH variations.

The need and importance is increasingly felt for altering tumor microenvironment in order to block malignant cell growth and for example by increasing the clinical potential of T cell-based immunotherapy in cancer patients.

The object of the present invention is therefore the development of improved compositions and their use in overcoming immune escape in antineoplastic therapy, chronic inflammatory diseases associated with an acidic microenvironment and in chronic infection thereto associated.

### Summary of the invention

The present invention concerns a proton pump inhibitor for use as an immunomodulator.

For the purposes of the present invention, said proton pump inhibitor is selected from the group consisting of omeprazole, lansoprazole, pantoprazole, esomeprazole, dexlansoprazole, rabeprazole, tenatoprazole, or a mixture thereof, as indicated in the following detailed description. (E' possible estendere a molecole con analoghe attività ma non ancora commercializzate?) Advantageously, in a further embodiment, the present invention relates to a vaccine composition comprising an effective amount of a proton pump inhibitor and pharmaceutically acceptable excipients.

As will be further described in the detailed description of the invention, the vaccine composition of the present invention comprising at least one proton pump inhibitor has the advantage of being useful in the treatment or prevention of a cancerous condition or a tumor or in the treatment of a chronic infection.

### Brief description of the drawings

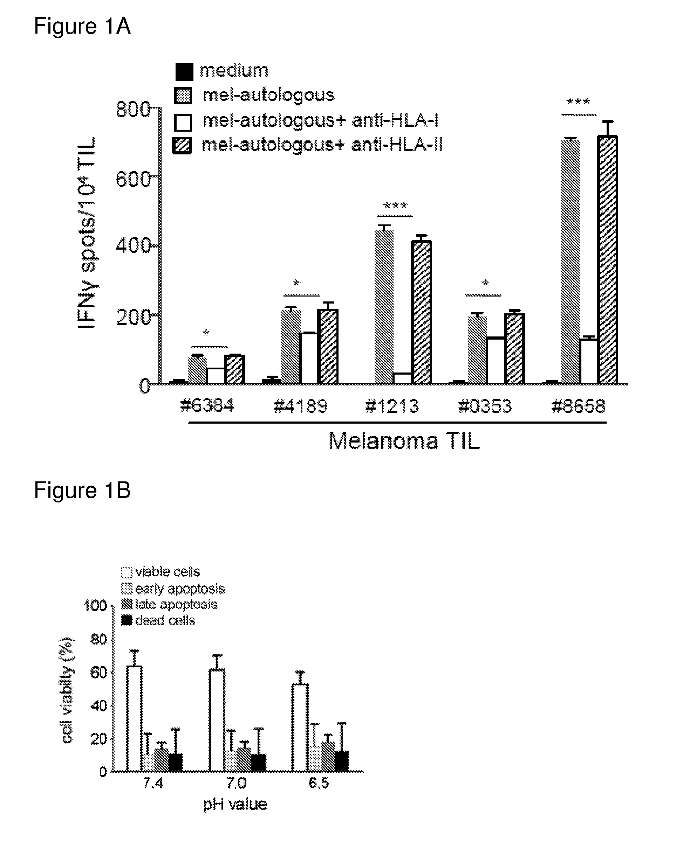

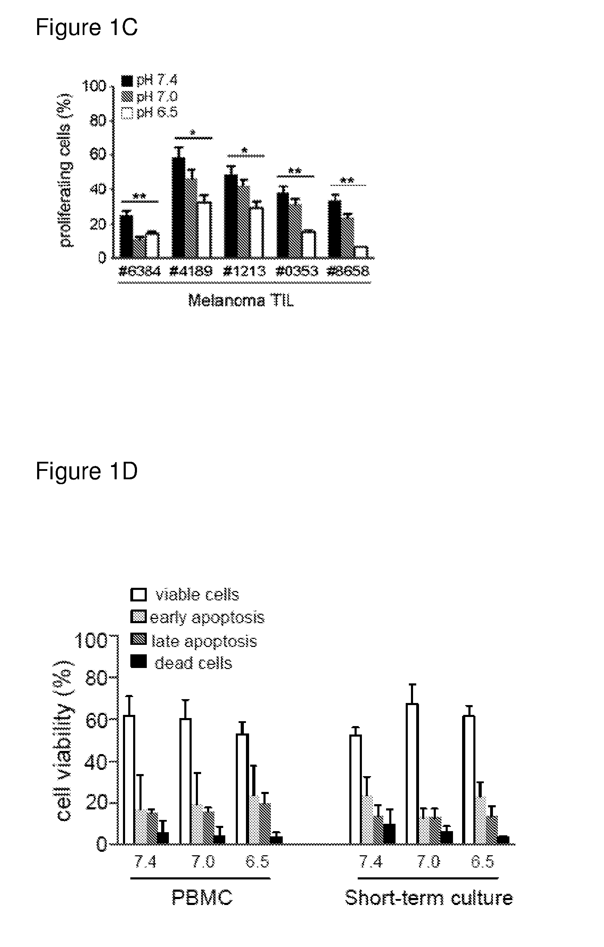

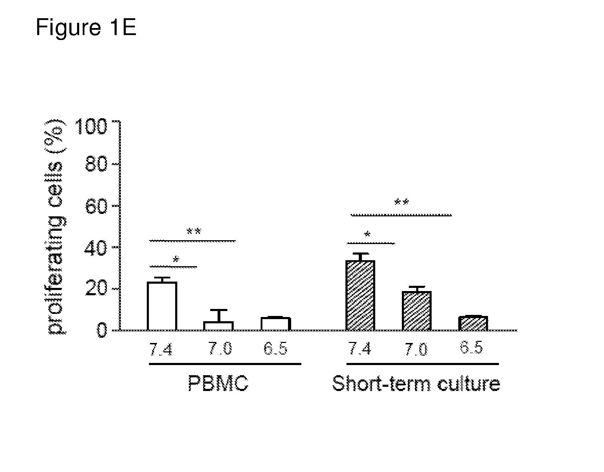

The characteristics and advantages of the present invention will be apparent from the detailed description reported below, from the Examples given for illustrative and non-limiting purposes, and from the annexed Figures 1-11, wherein:
**Figure 1****:** shows Low pH induces a defect in TIL proliferation without affecting cell viability. (A) Tumor specificity of the TIL lines included in the study. TILs derived from stage IV melanoma patients were evaluated by IFN_{Y} ELISpot for the ability to recognize autologous tumor cells in a HLA-restricted fashion. Targets were pretreated or not with anti-HLA class I and anti-HLA class II mAbs to assess T cell receptor involvement. *.05<p<.01; **.01<p<.001;***p<.001 (Student's t-test) with respect to recognition of the same target in the absence of anti-HLA class I or anti- HLA class II mAbs. (B) Melanoma TILs (N₌5) were cultured for 3 days in complete medium at the indicated pH values and then evaluated for viability by PI/Annexin V staining. Data represent the mean±SD. (C) The same TILs were also tested for their proliferative activity in the presence of CD3/CD28 T Cell Expander beads (TIL:beads ratio = 20:1) using the intracellular fluorescent dye CFSE. Data represent the mean±SD of triplicate determinations. *p<.05; **.01 <p<.001 (Student's t-test). (D) PBMCs from healthy donors (N=5) or short-term (14 days) MelanA/MART-1-specific T cell cultures (N₌5) generated from melanoma patients' PBMCs by in vitro peptide sensitization and 60 IU/ml IL2 were cultured for 3 days in complete medium at the indicated pH values (horizontal line). Cells were then evaluated for viability by a PI/AnnexinV assay. (E) Cells from the same cases were also tested for their proliferative activity in the presence of CD3/CD28 T Cell Expander beads (T cells:beads ratio = 20:1) using the intracellular fluorescent dye CFSE. Data represent the mean±SD of the tested samples. * p<.05; **.01 <p<.001 (Student's t-test).

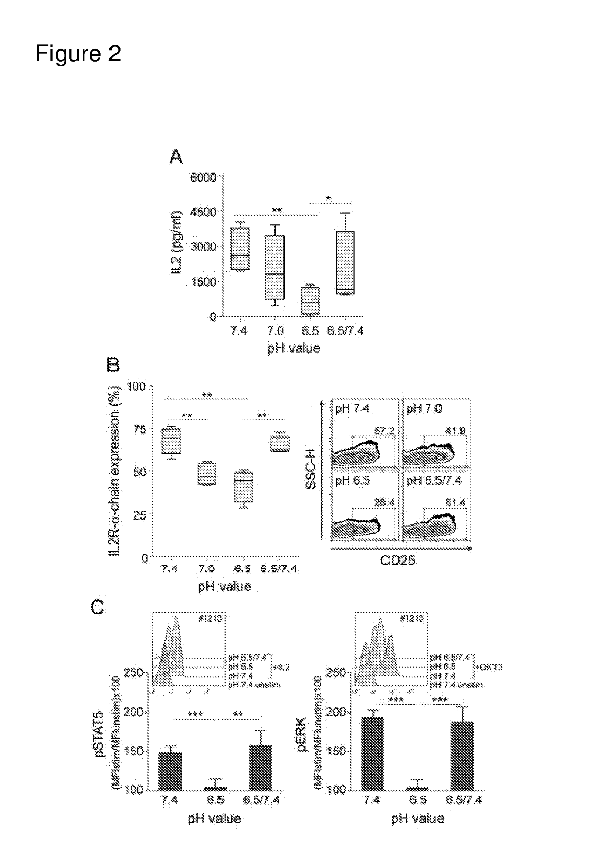

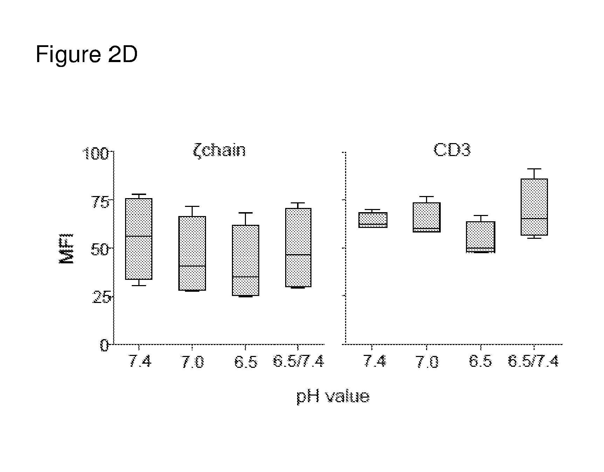

**Figure 2****:** shows Low pH induces a deficit in IL2 secretion and IL2R α-chain (CD25) expression and impairs the levels of STAT5 and ERK activation in melanoma TILs.
   Melanoma TILs were cultured in complete medium at the indicated pH values. At day 3 lymphocytes were (A-B) stimulated with CD3/CD28 T Cell Expander beads (TIL: beads ratio = 20:1) to asses IL2 production (A) and CD25 expression (B). Bars, box-and whisker diagrams (N=5). Horizontal line, median. As a representative example, TIL dot plots from case #0353 are shown with numbers indicating the percentage of cells expressing IL2R (B, right panel). (C) The same TILs were evaluated by flow cytometry for STAT5 and ERK activation in response to IL2 (150 ng/ml, left panel) or OKT3 (2 µg/ml, right panel). As representative example, TIL histogram plots from case #1213 are shown. Data represent the mean ± the SD. In all experiments, TILs restored to pH 7.4 for 24h before the assay (6.5/7.4 in A-C) were also included in the analysis. *0.05<p<0.01; **.01<p<.001;***p<.001 (Student's t-test). (D) Down-modulation of the ζchain and CD3 expression in TILs cultured at low pH: Melanoma TILs were cultured for 3 days in complete medium at the indicated pH values and then evaluated for their expression of ζchain and CD3 by flow cytometry. TILs restored to pH 7.4 for 24h before the assay were also included in the analysis (6.5/7.4). Bars, box-and-whisker diagrams (N=5). Horizontal line, median. MFI, mean fluorescence intensity.

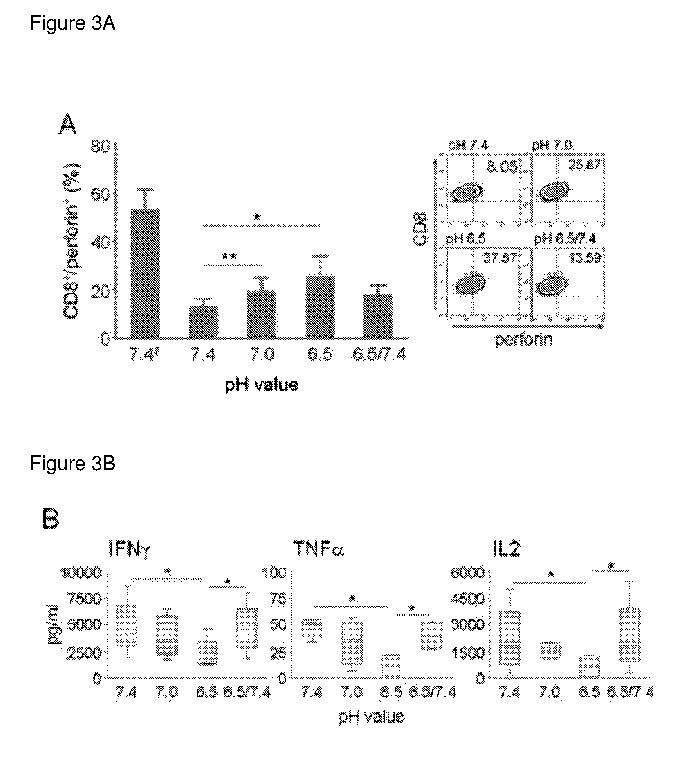

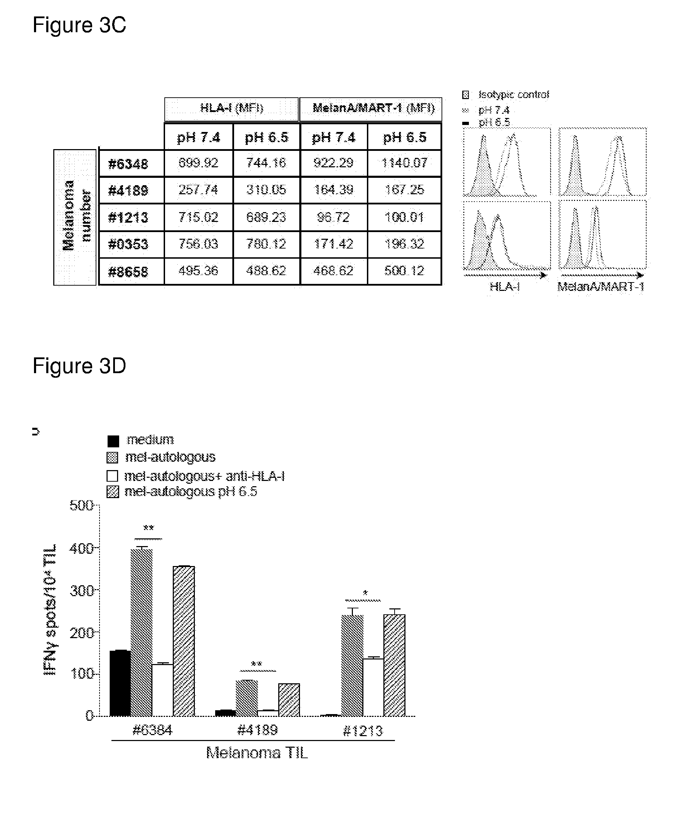

**Figure 3**(A) and Figure 3 (B) respectively show Low pH compromises the effector function of melanoma TILs. (A) Melanoma TILs (N=5) were cultured in complete medium at the indicated pH values. At day 3 lymphocytes were stimulated with autologous tumor cells (TIL:tumor ratio = 8:1) and tested for perforin release by intracellular staining. Data represent the mean ± the SD. $, TILs cultured with medium alone. As representative example, TIL dot plots from case #8658 are shown with numbers indicating the percentage of CD8+/perforin+ T cells (upper right panel). (B) IFN_{Y}, TNFα and IL2 release by TILs after a 24h co-incubation with autologous tumor cells (TIL:tumor ratio = 8:1). Bars, box-and-whisker diagrams (N=5). Horizontal line, median. In all experiments, T cells restored to pH 7.4 for 24h before the assay were also included in the analysis (6.5/7.4 in A and B). *p<.05; **.01<p< .001 (Student's t-test). Short-term culture of melanoma cell lines at low pH does not affect specific immune recognition by autologous TILs: (C) Melanoma cell lines were cultured in complete medium for 24h at the indicated pH values and then tested for their expression of HLA class I and MelanA/MART-1 antigens by flow cytometry. MFI, mean fluorescence intensity. In the right panel, histogram plots of two representative cases are shown (#6348, #4189). (D) TILs from stage IV melanoma patients were evaluated by IFN_{Y} ELISpot for the ability to specifically recognize autologous tumor cells maintained for 24h in medium buffered at pH 6.5. Targets were pre-treated or not with anti-HLA class I mAb to assess T cell receptor involvement. *.05<p<.01;**.01<p<.001 (Student's t-test) with respect to recognition of the same target in the absence of anti-HLA class I mAb.

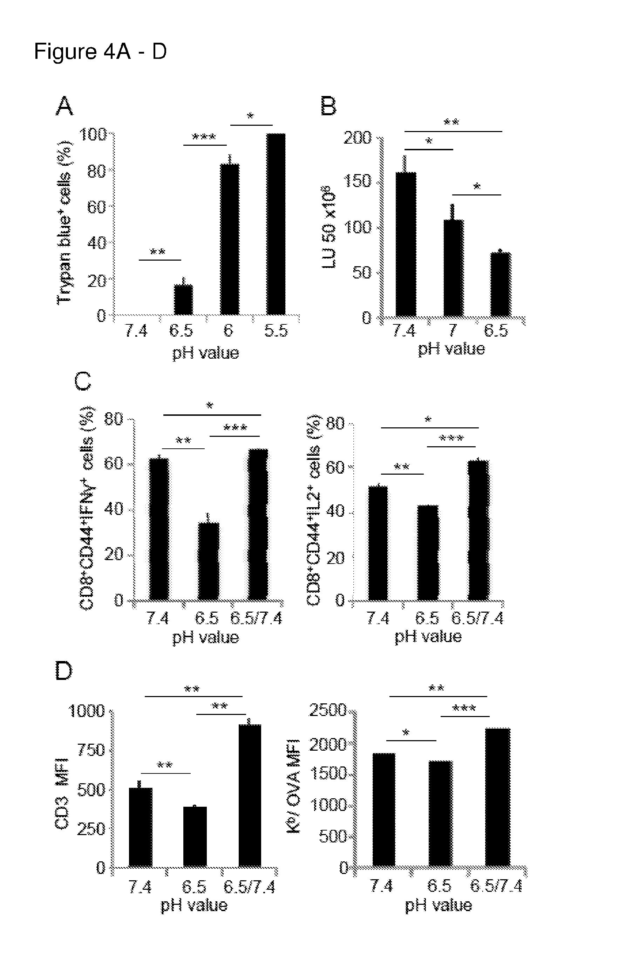

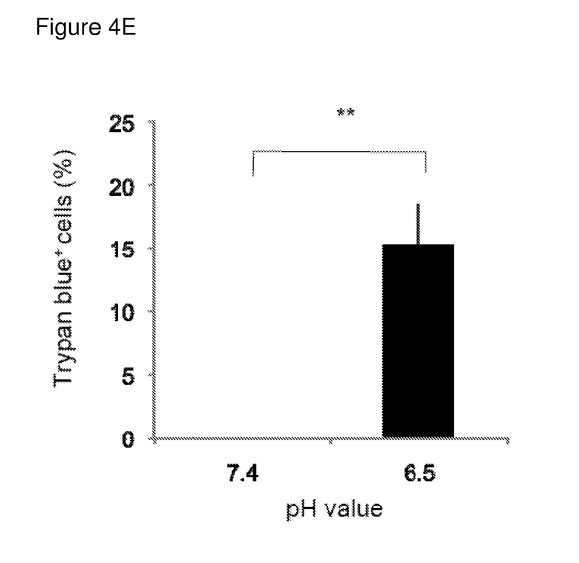

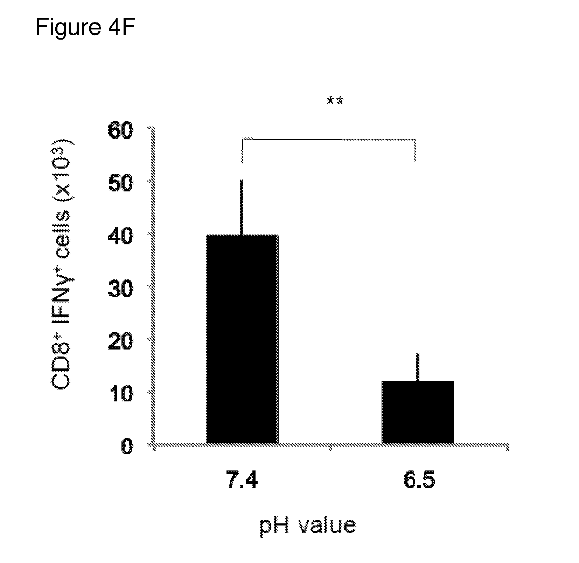

**Figure 4** Low pH also induces reversible anergy in mouse T lymphocytes. Activated TCR transgenic OTI cells, specific for the antigen oavlbumin (OVA), were cultured in complete medium at the indicate pH values and assessed for viability by trypan blue exclusion (A) or cytotoxic activity in a standard ⁵¹Cr release assay (B). Data are reported the mean±SD of trypan blue+ cell counts or as the average lytic unit 50 (LU 50x106 cells). (C) The same OTI cells were assessed for IFN_{Y} (left panel) and IL2 (right panel) production by intracellular staining. Data represent the percentage of CD8+CD44+IFNy+ and CD8+CD44+IL2+ T cells. (D) The level of CD3 (left panel) and TCR (right panel) expression on CD8+ T cells was assessed by flow cytometry. MFI, mean fluorescence intensity. Lymphocytes restored to pH 7.4 for 24h before the assay were also included in the analysis (6.5/7.4 in C-D). Results are representative of at least three independent experiments. ***p<.001; _{**}.001<p<.01; *.01<p<.05 (Student's t-test). Low pH impairs in vitro restimulation of in vivo-activated T cells.
   Mice were immunized by one i.d. injection of dendritic cells (DC) pulsed with the epitope peptide STEAP186-193. One week later, splenocytes from vaccinated animals were restimulated in vitro in the presence of STEAP186-193. Five-day blasts were either assessed for viability by trypan blue exclusion
   (E) or for IFN_{Y} production against STEAP by flow cytometry (F). Data are reported as the mean percentage±SD of trypan blue+ cells (E) or as the
   number of IFNy+ cells within the CD8+ T cell population (F). Data are representative of at least two independent experiments. **.001<p<.01 (Student's t-test).

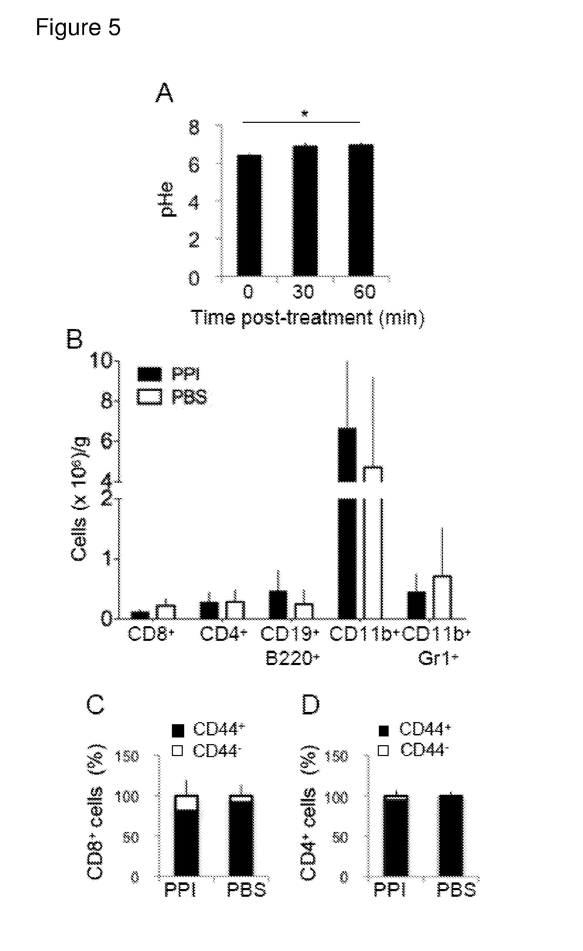

**Figure 5****:** Characterization of the melanoma microenvironment before and after PPI treatment. (A) Tumor extracellular pH (pHe) was measured by MRS in animals engrafted with B16 cells expressing OVA (B16-OVA; 2x105 cells; N=6) before (0 min) and 30 and 60 min after i.p. infusion of PPI (12.5 mg/Kg). Data are reported as the mean±SD. *.01<p<.05 (Student t-test). (B-D) 7-day B16-OVA melanoma-bearing mice received DC pulsed with the epitope peptide OVA257-264 (5x105 cells i.d), followed by i.p. administration of PPI (N₌5) or PBS (N₌5). Tumor cell suspensions obtained after 24h were analyzed for expression of the indicated antigens by flow cytometry (B). Data are reported as the number of positive cells ±SD. The mean percentage ±SD of CD8+CD44+ (C) and CD4+CD44- (D) T cells is also reported. Each panel is representative of three independent experiments.

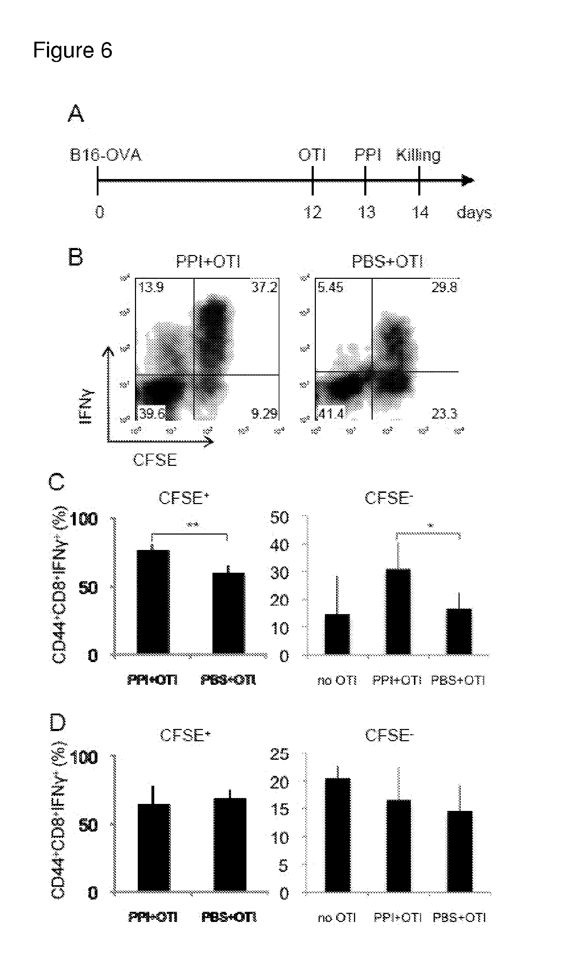

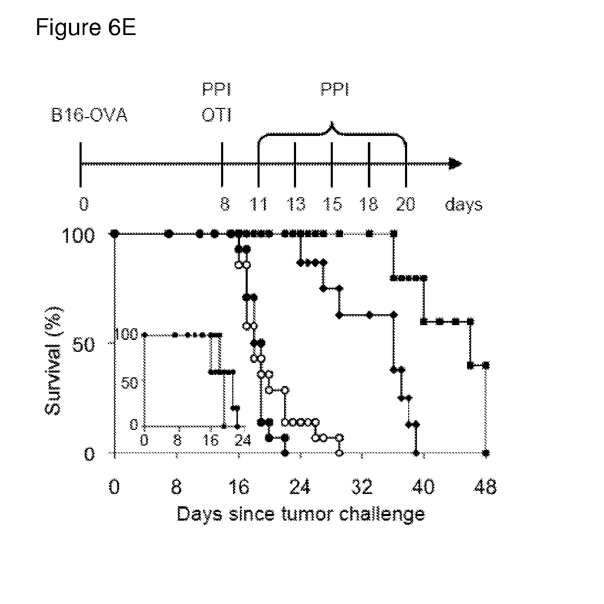

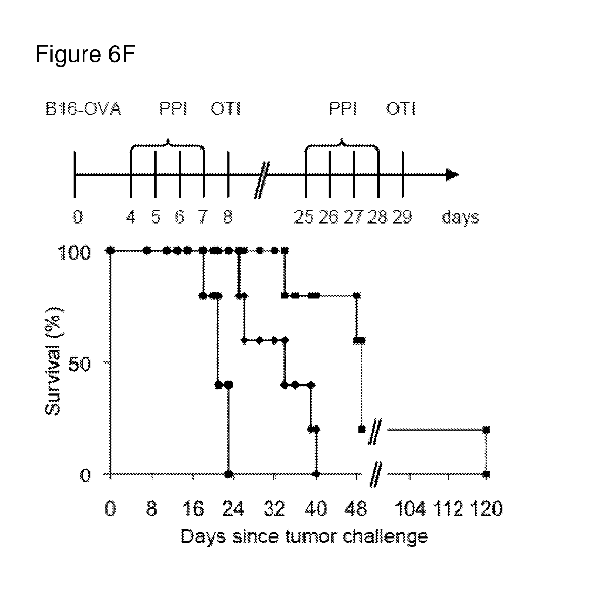

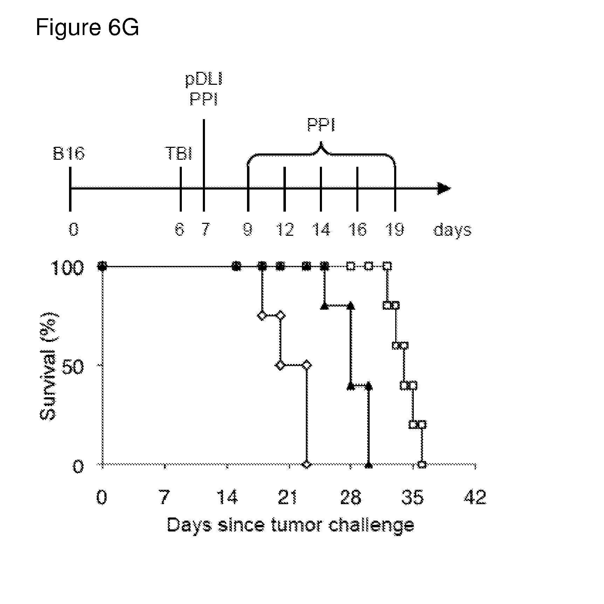

**Figure 6****:** PPI treatment improves TIL effector function. (A) Schematic representation of the experiment. Mice were challenged s.c. with B16-OVA cells and 12 days later were infused with activated CFSE-labelled OTI cells (3x107). One day after infusion animals were treated i.p. with esomeprazole (PPI+OTI; N=5) or PBS (PBS+OTI; N=5). The next day, tumor cell suspensions were assessed for intracellular cytokine release by flow cytometry. As a control, a group of mice were included that did not receive adoptive transfer (no OTI; N=3). (B) As a representative example, TIL dot plots (gated on CD8+CD44+ cells) are shown with numbers indicating the percentage of cells in each quadrant. (C) The percentage of CD44+CD8+IFN-Y+ T cells within the CFSE+ (left panel) and CFSE- (right panel) populations. (D) The percentage of CD44+CD8+IFNy+-CFSE+ (left panel) and -CFSE-(right panel) populations in the spleen of tumor-bearing mice. Data are representative of at least three independent experiments. **.001<p<.01; *.01<p<.05 (Student's t-test).(E)PPI treatment increases the therapeutic potential of adoptive immunotherapy. 8-day B16-OVA or B16 melanoma bearing-mice were randomly assigned to either one of the treatment groups described in A, B and C (5-14 animals/group). (F) Treatments: PBS (black circles); PPI (open circles); activated OTI cells (6x106/mouse; black diamonds), or PPI+OTI (black squares). Kaplan-Meyer plot log-rank tests: PBS vs. PPI p=.459; PBS vs. OTI p<.0001; PBS vs. PPI+OTI p<.0001; PPI vs. OTI p<.0002; PPI vs. PPI+OTI p<.0002; and OTI vs. PPI+OTI p<.001. (Insert in B) As control, PPI+OTI (black squares) treatment was performed in mice affected by B16 melanoma that did not express the OVA antigen; PBS (black circles). (G) Treatments: PBS (black circles); OTI cells (black diamonds), or PPI+OTI (black squares). Kaplan-Meyer plot log-rank tests: PBS vs. OTI .01<p<.001; PBS vs. PPI+OTI .01<p<.001; and OTI vs. PPI+OTI .05<p<.01. (G) Treatments: total body irradiation (TBI; 600 rad) in combination with PPI (open diamonds); TBI plus adoptive transfer of splenocytes from mice pre-sensitized against TRP-2 (60x 1 06/mouse; pDLI, black triangles); or TBI+pDLI+PPI (open squares). Kaplan-Meyer plot log-rank tests: TBI+PPI vs. TBI+pDLI p=.004; TBI+PPI vs. TBI+pDLI+PPI p₌.0026; and TBI+pDLI vs. TBI+pDLI+PPI p₌.004.

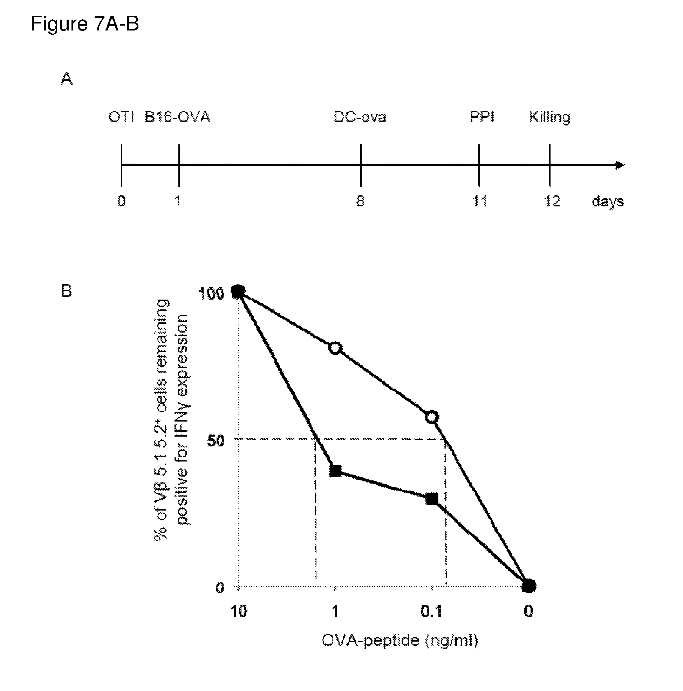

**Figure 7****:** PPI treatment favors high-functional avidity of CD8+ T cells. (A) Schematic representation of the experiment. Mice were adoptively transferred with 12x 106 naïve OTI cells. One day later, they were challenged s.c. with B16-OVA cells. Seven days later (day 8), mice were vaccinated with DC-OVA257-264, and after 3 additional days (day 11) mice were treated i.p. with PBS (black squares) or PPI (open circles). Animals were sacrificed 24h after the last treatment. (B) CD8+ T cells (OTI and endogenous) were magnetic-bead sorted from tumor cell suspensions and assessed for intracellular cytokine production in the presence of RMA cells either pulsed or not with increasing concentrations of OVA257-264 peptide. Data are representative of two independent experiments.

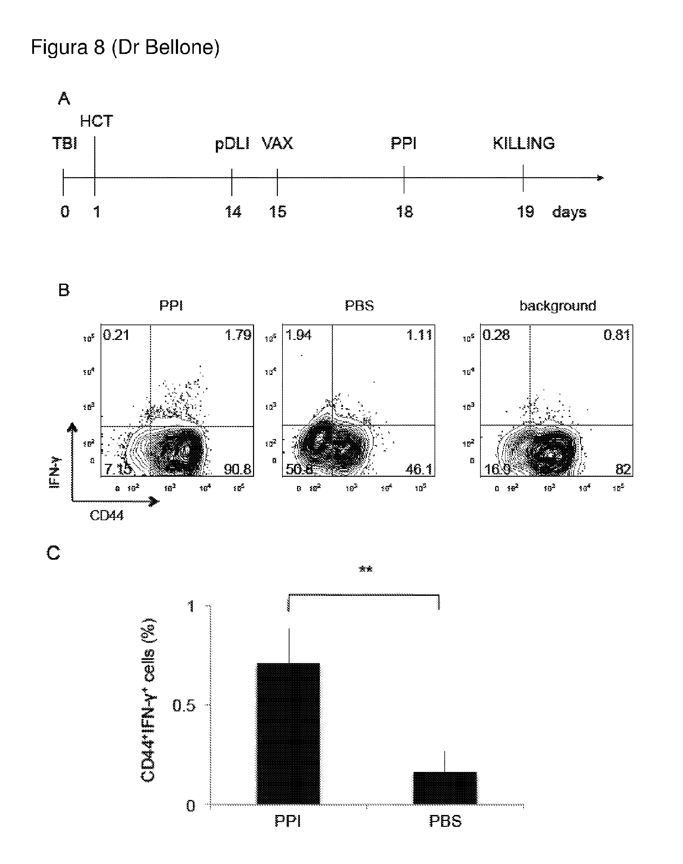

**Figure 8****:** PPI treatment increases the functional activity of prostate infiltrating tumor--specific T cells. (A) schematic representation of the experiment. Transgenic adenocarcinoma of the mouse prostate (TRAMP) mice (3 mice/group) were subjected to TBI and a day later transplanted with HSCT (1 x 107). Two weeks later the HSCT, mice received a pDLI of 6 x 107 splenocytes derived from congenic females previously sensitized against male antigen. A day later the DLI, mice were vaccinated with DC-Tag-IV and treated i.p. with PPI (0,25 mg/kg) or PBS (see text). One day later prostate tumor cell suspensions were analyzed ex vivo by flow cytometry for IFN-γ intracellular secretion following Tag-IV stimulation, (B, left and middle panel). As control, cells were left unstimulated (B, right panel). Representative dot plots are shown after gating on viable CD8+TIL. Numbers refer to the percentage of cells in each quadrant. (C) Quantification of CD44+IFN-y+ cells within the viable CD8+ cells is reported. **0.001 < p < 0.01.

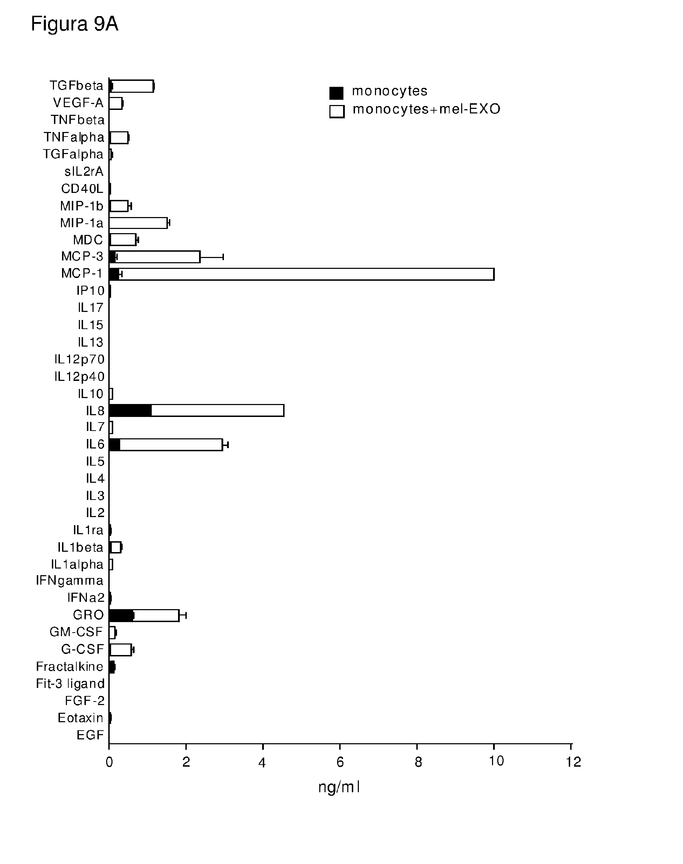

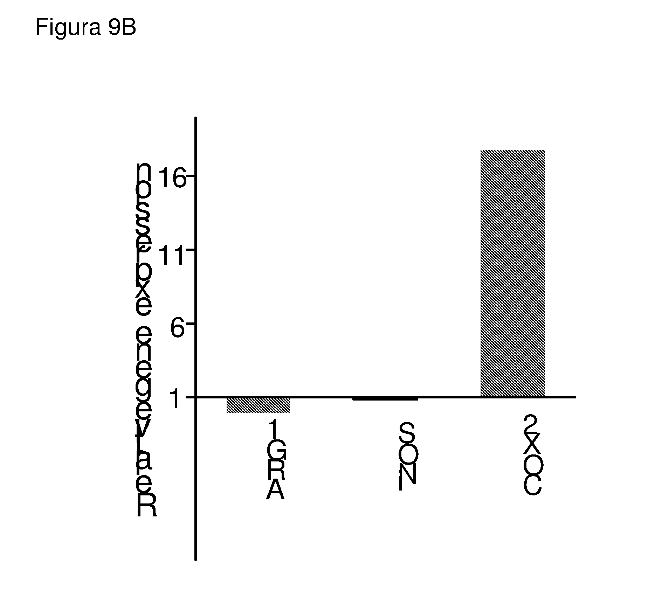

**Figure 9****:** Cytokine induction in primary human monocytes from healthy donors (HD) peripheral blood mononuclear cells (PBMC) by melanoma-derived exosomes (mel-EXO). Primary human monocytes from HD PBMC were co-incubated with mel-EXO (100 µg) for 24 hours and evaluated for (A) the cyto/chemokines release using MILIPLEXTM Human Cytokine/Chemokine panel coupled with the Luminex®xMAP® platform (Bars, mean±SD), or(B) the gene expression levels of Arginase 1 (ARG1), inducible nitric oxide synthase (iNOS) and cyclooxigenase 2 (COX2). Quantification was performed by real-time PCR (qRT-PCR). The relative mRNA expression was expressed as 2^{-ΔΔCT}, wherein ΔCT=CT target genes-CT GAPDH, and ΔΔCT=ΔCT sample-ΔCT calibrator (monocytes sample). Data analysis was done using the SDS version 2.1 software.

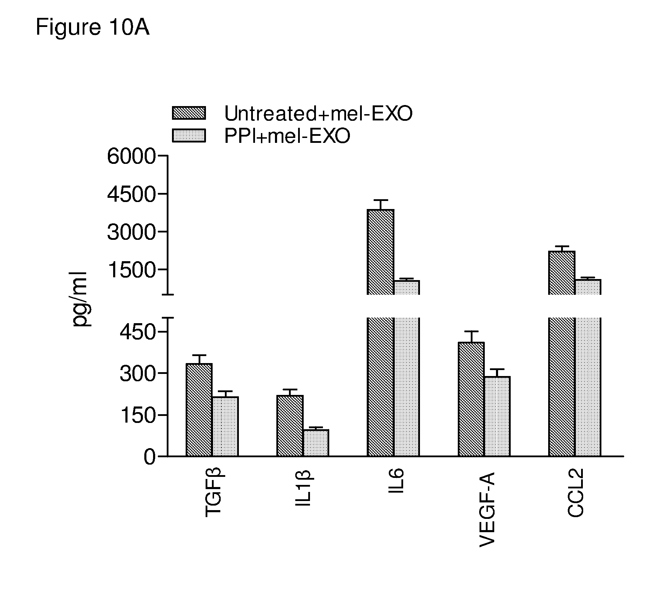

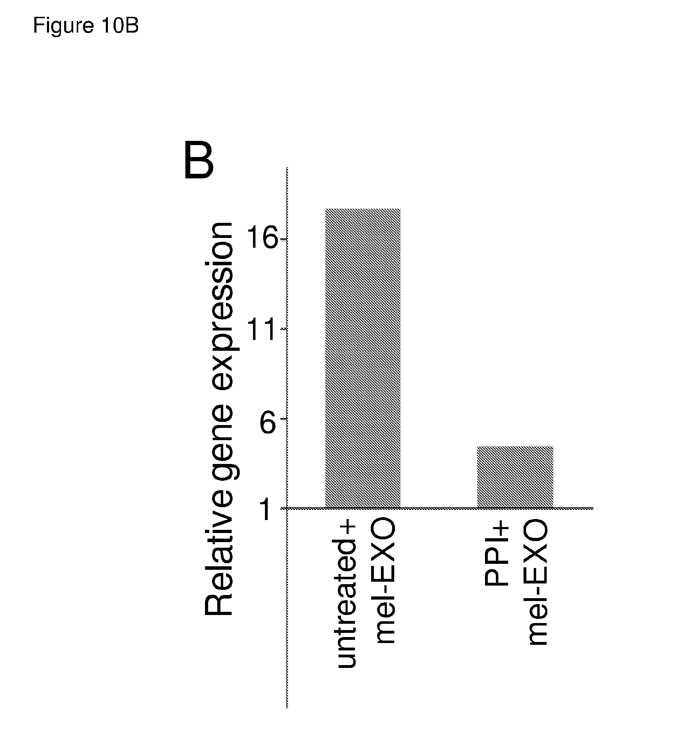

**Figure 10****:** Cytokine modulation in primary human monocytes from HD PBMC by mel-EXO. Primary human monocytes from HD PBMC were pre-treated (3 hours) or not with esomeprazole (100 µg/ml), and then co-incubated with mel-EXO (100 µg) for 24 hours and evaluated for (A) the cyto/chemokines release using FlowCytomix assay (Bender MedSystem) (Bars, mean±SD), or for (B) the gene expression levels of COX2. Quantification was performed by real-time PCR (qRT-PCR). The relative mRNA expression was expressed as 2^{-ΔΔCT}, wherein ΔCT=CT target genes-CT GAPDH, and ΔΔCT=ΔCT sample-IiCT calibrator (monocytes sample). Data analysis was done using the SDS version 2.1 software.

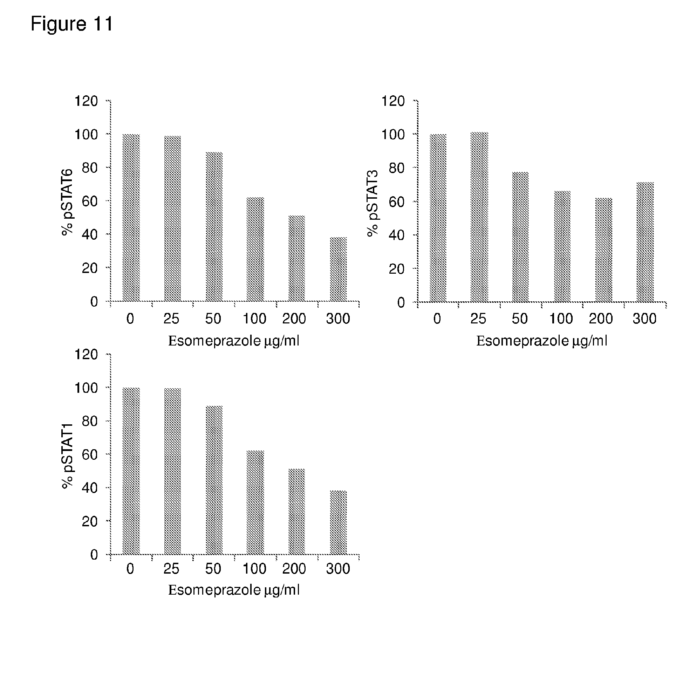

**Figure 11****:** pSTAT evaluation in primary human monocytes from HD PBMC. Primary human monocytes from HD PBMC were pre-treated (3 hours) with esomeprazole at the indicated doses, stimulated with the appropriate cytokine (30 min) and then evaluated for the STAT phosphorylation state.

### Detailed description of the invention

The present invention concerns a proton pump inhibitor (PPI) for use as an immunomodulator.

According to a preferred aspect, the proton pump inhibitor of the present invention is a 2- pyridyl methylsulphinyl benzimidazole proton pump inhibitor.

PPIs are a group of drugs which have been developed to cure gastric acid related diseases, and whose main action is a pronounced and long-lasting reduction of gastric acid production. From the structure point of view, they are substituted 2-pyridyl methylsulphinyl benzimidazoles that share a similar core structure.

According to a more preferred aspect, the PPI according to the present invention is selected from the group consisting of omeprazole, lansoprazole, pantoprazole, esomeprazole, dexlansoprazole, rabeprazole, tenatoprazole or a mixture thereof.

A preferred PPI is esomeprazole.

The therapeutic efficacy of PPIs is guaranteed by their ability of inhibiting the activity of the hydrogen/potassium adenosine triphosphatase enzyme system (H⁺/K⁺- ATPase or proton pump).

A further aspect of the present invention relates to a PPI, for use in the combination therapy or prophylaxis of a disease condition, wherein the PPI is administered prior to at least one drug indicated against said condition. Combination therapy or polytherapy is the use of more than one medication or other therapy. Most often it consists in simultaneous administration of two or more medications to treat a single disease. Combination therapy may be achieved by giving separate drugs, or, where available, by giving combination drugs, which are dosage forms that contain more than one active ingredient. Conditions treated with combination therapy include tuberculosis, leprosy, cancer, malaria, and HIV/AIDS. A major benefit of combination therapies is that they can avoid the development of drug resistance, due to the fact that a pathogen or tumor is less likely to have resistance to multiple drugs simultaneously.

In a preferred aspect, the present invention relates to a PPI, for use in the combination therapy or prophylaxis of a disease condition, wherein said condition is a cancerous condition.

In a further preferred aspect, the present invention relates to a PPI, for use in the combination therapy or prophylaxis of a disease condition, wherein said condition is a tumor.

A cancerous condition, or cancer is a disease in which a group of cells display uncontrolled growth, invasion that intrudes upon and destroys adjacent tissues, and sometimes metastasis, or spreading to other locations in the body via lymph or blood. These three malignant properties of cancers differentiate them from benign tumors, which do not invade or metastasize.

To date all solid tumors are characterized by different levels of acidosis. These include breast, prostate, colon, pancreatic, gastric, esophageal, thyroid, ovarian, uterine, bladder and lung cancers, osteosarcomas and soft tissues sarcomas, lymphomas, glioblastomas and neuroblastomas. However also bone marrow blasts from acute lymphoblastic leukemias patients have shown to be sensible to PPI treatment.

In a further aspect, the present invention relates to a PPI, for use in blocking or reducing tumor growth, through anti-tumor immune response.

PPIs have been advantageously seen to buffer the pH of the tumor site, which due to abnormal glycolysis, high lactic acid production, proton accumulation and a reversed intra-extracellular pH gradient make the tumor site a hostile microenvironment for cells other than cancer cells. pH values most frequently detected within a tumor mass are in the acidic range, most often in the range of pH 6-6.5.

PPIs advantageously buffer the pH to physiologic values and allow the complete recovery of T cell function. Furthermore, systemic treatment with proton pump inhibitors (PPls) transiently corrected the intra-tumor acidity and favoured tumor-infiltrating T lymphocytes (TIL) proliferation and IFN_{Y} secretion following either active or adoptive immunotherapy.

PPI treatment according to the present invention was seen to increase the therapeutic efficacy of adoptively-transferred T cells. Therefore, the acidity of the tumor microenvironment represents a novel mechanism of tumor immune escape that can be overcome by PPIs, to increase the clinical potential of T cell-based immunotherapies in cancer patients.

Local acidity plays a crucial role in impairing T cell responses and promoting immunosuppression related to chronic inflammation. In this regard, high dose PPI administration can significantly block these effects by locally buffering acidic pH and interfering with negative effects of tumor-associated chronic inflammation. PPI treatment could be beneficial in suppressor cells and other acidity-activated microenvironment components.

PPI according to the present invention therefore promote specific tumor immunity through the buffering of tumor microenvironment acidity, and also impact on one the major immunosuppressive pathways displayed by cancer patients, i.e. the activation and accumulation of myeloid-derived suppressor cells (MDSC).

In a further preferred aspect, the present invention relates to a PPI for use in the combination therapy or prophylaxis of a disease condition, wherein said condition is a chronic infection or wherein said condition is a chronic infection associated with a cancerous condition or a tumor.

PPIs reduce the detrimental effect of chronic inflammation associated with cancer or other diseases, by specifically reducing the recruitment of myeloid cells. PPIs have been seen to further improve specific immune responses by reducing the immunosuppressive impact of myeloid-derived suppressor cells and other acidity-activated microenvironment components.

PPIs according to the present invention have advantageously been used in potentiating immune responses, either spontaneous or induced by active or adoptive immunotherapy, which is both useful in cancerous conditions or in tumors and chronic infections, or when a chronic infection is associated with a cancerous condition or a tumor. PPIs have a beneficial effect of pH buffering and for example in the therapeutic activity of passive immunotherapy based on antibody (Ab) administration.

PPI also reduce exosome release by cancer cells, thus reducing the level of spread of tumor-derived exosome both into the tumor microenvironment and at the systemic level.

Preferably, the present invention relates to a PPI, for use in a chronic infection, wherein said chronic infection is tuberculosis or AIDS, as well as chronic inflammatory diseases where an acidic microenvironment is associated with alternatively activated macrophages and myeloid derived cells with a profibrotic and pro-angiogenic phenotypes (e.g., sarcoidosis, peripheral spondyloarthritis, psoriasis)

Advantageously, in a further embodiment, the present invention relates to a vaccine composition comprising an effective amount of a proton pump inhibitor and pharmaceutically acceptable excipients.

In a preferred embodiment, the present invention relates to a vaccine composition, wherein said vaccine is a cancer preventive (or prophylactic) vaccine or a cancer treatment vaccine.

A preventive (or prophylactic) vaccine, is intended to prevent cancer from developing in healthy people, while a treatment (or therapeutic) vaccine, is intended to treat an existing cancer by strengthening the body's natural defences against the cancer.

Cancer treatment vaccines are therefore designed to treat cancers that have already developed. They are intended to delay or stop cancer cell growth; to cause tumor shrinkage; to prevent cancer from coming back; or to eliminate cancer cells that have not been killed by other forms of treatment. Advantageously, the vaccine composition according to the present invention is a peptide derivative, a protein or a DNA vaccine.

In a preferred embodiment, the present invention relates to a vaccine composition, for use in the treatment or prevention of a cancerous condition or a tumor or in the treatment of a chronic infection.

### EXAMPLES

### Example 1.

### Acidic pH induces reversible anergy in tumor-infiltrating T lymphocytes.

To test the effects of pH alterations on the proliferative and functional properties of tumor-specific T cells, TIL lines were used (prepared in our laboratory from metastatic melanoma lesions) (Dudley ME, Wunderlich JR, Shelton TE, Even J, Rosenberg SA.; J Immunother 2003;26:332-42), composed mostly of CD8+ cells (Table 1) and displayed HLA class l-mediated recognition of autologous melanoma cells (Fig. 1 A).

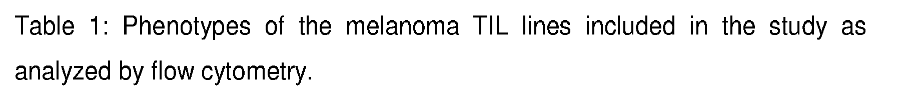

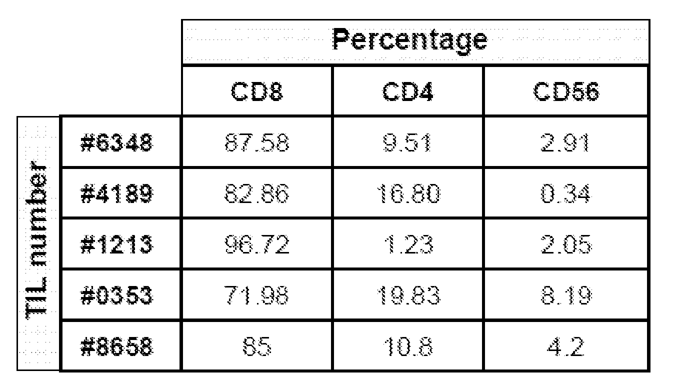

To mimic pH conditions at the tumor site, TILs were cultured at pH ranging from 7.4 to 6.5 for 3 days, as previous experiments showed that longer exposure was associated with significant apoptosis.

### Results

After 3 days of culture, Annexin-V/Pl staining showed that no major change in the percentage of early or late apoptotic cells was observed at pH 7.0 or 6.5 relative to pH 7.4 (Fig. 1 B). The proliferative activity of the TILs was then evaluated by CFSE dye dilution following CD3/CD28-stimulation.

Proliferation was significantly decreased at pHs lower than physiological levels in all cases, with a mean inhibition of 30% (with a range of 14-54%) and 56% (with a range of 40-90%) at pH 7.0 and 6.5, respectively (Fig. 1 C). Similar data were obtained using CD3+ T cells from healthy donors or Ag-specific CD8+ T lymphocytes from melanoma patients, suggesting this is a more general response of T cells to unfavorable pH conditions rather than a specific TIL feature (Fig. 1D and Fig.1E).

Impaired proliferation in the absence of apoptosis at acidic pH levels suggested the onset of anergy. Indeed, the ability to secrete IL2 in response to mitogenic stimuli, a feature typically associated with T cell anergy, was strongly affected in TILs maintained for 3 days at an acidic pH (Fig. 2A).

Interestingly, initial IL2 production was restored when TILs first cultured in preconditioned medium (pH 6.5), were then returned to physiologic pH (7.4) for 24h before the assay, suggesting that this phenomenon is reversible at least under experimental conditions (Fig. 2A). CD25 expression in response to CD3/CD28 stimulation was also progressively affected by culturing TILs at decreasing pH levels, and expression was similarly reversed by physiologic pH buffering for 24h (Fig. 2B). Anergic T cells may have compromised JAK3/STAT5 pathway signaling (24) and reduced activation of ERK (25). Correspondingly, STAT5 and ERK phosphorylation in response to IL2 or OKT3 activation, respectively, was completely abrogated in TILs following 3 days of culture at pH 6.5, while a full recovery was obtained by pH buffering for an additional 24h (Fig. 2C). A tendency to decrease CD3 and ζchain expression, another well-documented feature of T cells in cancer patients (26), was additionally observed upon TIL culturing at a low pH (Fig. 2D). TILs were also assessed for their activity against tumor cells in altered pH conditions. Perforin degranulation and IFN_{Y}, TNFa and IL2 release in response to autologous tumor cells were significantly impaired at pH 6.5, although these functions were restored with additional 24h incubation at physiological pH (Fig. 3A-C). The detrimental impact of low pH on T cell activity was not due to the effects of pH on the tumor target cells, since these cells did not change their surface expression of HLA-class I, tumor antigens (Fig. 3C), or the HLA/peptide complexes recognized by the Agspecific T cells under low pH conditions (Fig. 3D).

These data demonstrate that acidic pH conditions resembling those observed in tumor lesions promote a reversible anergic state in human TILs, probably due to down-regulation of the high affinity IL2 receptor (IL2R) and perturbation of the STAT5 and ERK pathways.

### Example 2.

### Effector functions of murine T lymphocytes are also affected by low pH.

To investigate the sensitivity of murine T lymphocytes to acidic pH, in vitro primed OTI cells (Boni A, lezzi G, Degl'lnnocenti E, Grioni M, Jachetti E, Camporeale A, et al.; Eur J Immunol 2006;36(12):3157-66) were cultured in medium at from pH 5.5 to 7.4 for an additional 24h and assessed for their viability.

### Results:

At pH<6.5 most of the cells died, whereas >80% survival was observed at pH 6.5 (Fig. 4A). Upon antigen stimulation, cytolytic activity, as well as IFN_{Y} and IL2 release (Fig. 4B-C), were substantially reduced. As for human TILs (Fig. 2 and 3), the low pH-associated reduction in cytokine secretion by OTI cells was reversed by pH buffering (Fig. 4C). CD3 and TCR expression were also significantly down-regulated in OTI cells exposed to pH 6.5 (Fig. 4D) and again fully recovered upon pH adjustment, corresponding with the trend in TCR ζchain/CD3 down-modulation in human TILs exposed to an acidic pH (Fig. 2D).

Acidity mediated functional impairment could be reproduced as well in spleen T cells from mice sensitized against the natural tumor antigen STEAP (Fig. 4E and 4F).

### Example 3.

### Treatment with high-dose esomeprazole mediates buffering of tumor pH and improves TIL effector functions.

To investigate whether tumor pH buffering could result in improved T cell function *in vivo,* we first evaluated the extracellular pH (pHe) of tumor masses by magnetic resonance spectroscopy (MRS) in mice challenged subcutaneously (s.c.) with B16-OVA melanoma cells. Mice were then treated with a high dose of esomeprazole (12.5 mg/kg), a PPI used as a standard therapy for neutralization of gastric acid.

### Results:

The impact of PPI treatment on tumor pHe was evaluated. At baseline, the tumor pHe was approximately 6.5, while PPI therapy caused a rapid increase in the tumor pHe, which reached 7.0 within 60 min (Fig. 5A). Mice bearing 7-day-old melanomas were then vaccinated with DC-OVA257-264 and treated at the peak of the vaccine-induced immune response (i.e., day 3 post vaccine) with either PBS or PPI.

Mice were sacrificed after an additional 24h when tumor lesions were approximately 8 mm² in size. The leukocytes infiltrating the tumor mass were predominantly macrophages (CD11b), and to a lesser extent CD8+ and CD4+ T cells, B cells (CD19+B220+) and immature myeloid-derived cells (CD11b+Gr1+) (Fig. 5B). Most CD4+ and CD8+ T cells were CD44+, confirming exposure to antigen (Fig. 5C-D). Treatment with PPI did not substantially modify the inflammatory infiltrate in this experimental setting (Fig. 5B-D). As the limited number of CD8+ T cells infiltrating the tumor mass did not allow a functional evaluation of the tumor-specific T cells, mice bearing a 12-day-old melanoma were infused with activated CFSE-labeled CD44+ OTI T cells, treated after 24h with PPI or PBS and sacrificed 1 day later (Fig. 6A).

As shown in Figure 6B, the frequency of CFSE+ OT-1 cells was comparable in PPI- and PBS-treated mice. However, the percentage of CD44+CD8+IFN_{Y}+ T cells was significantly enhanced in tumor-derived CFSE+ T cells isolated from PPI-treated mice (Fig. 6C, left panel).

Interestingly, higher IFN_{Y} production was also detected in CFSE- TILs from PPI-treated mice, suggesting a beneficial effect of pH buffering on endogenous T cells as well (Fig. 6C, right panel).

Since PPIs are pro-drugs activated by protonation at low pH, their effect on resident lymphocytes should be selectively triggered by acidic environments, such as tumor lesions (Fig. 5A). Indeed, IFN_{Y} production by CD8+CD44+ T cells in both CFSE+ and CFSE- subsets isolated from the spleen, lung and kidney did not differ between the PPI- and PBS-treated groups (Fig. 6D). The in vivo effects of PPI treatment on TILs were also assessed in a model of active immunotherapy by adoptively transferring naïve OTI cells to mice and challenging them with B16-OVA cells 24h later. At day 8, animals were vaccinated with DC-OVA257- 264, treated with PPI at day 11 and then sacrificed 1 day later for TIL analysis (Fig. 7A).

As reported in Figure 7B, treatment with PPI increased the ability of magnetic bead sorted CD8+ T cells, including OTI and endogenous CD8+ T cells, to recognize OVA257- 264 by at least one log.

### Example 4.

PPI treatment increases the therapeutic potential of adoptive immunotherapy.

To investigate the therapeutic potential of the combination of PPI treatment and immunotherapy, mice bearing an 8-day melanoma were treated with 12.5 mg/kg esomeprazole, which in dosing experiments (ranging from 1.25-600 mg/kg) was the highest tolerated dose (Fig. 6E).

PPI treatment was repeated as in Figure 1A. Activated OTI cells were administered in a single treatment to melanoma bearing mice and displayed the best anti-tumor effect in the range of 1-6x10⁶ cells (Fig. 6E).

Combined treatment with PPI and OTI cells at the doses mentioned above induced a statistically significant increase in animal survival, with a doubling in the overall survival rate relative to mice treated with PBS alone (Fig. 6E). Notably, equal tumor growth inhibition was achieved when PPI was administered before OTI adoptive transfer (Fig. 6F).

The specificity of the treatment was confirmed by the finding that OTI cells and PPI treatment did not impact the survival of mice with B16 melanomas not expressing OVA (Fig. 6E, inset). To test whether pH tumor buffering could improve the efficacy of non-transgenic T cells, splenocytes from mice previously sensitized against tyrosinase-related protein 2 [TRP-2; (18)] were adoptively transferred into B16 melanoma-bearing mice. Prior to transfer, the mice were subjected to nonmyeloablative total body irradiation (TBI; 600 Rad) to favor in vivo expansion of the adoptively-transferred T cells (2). PPI treatment was applied as described in the treatment schedule (Fig. 6G). Survival was significantly prolonged in mice that received both T cells and PPI treatment compared to mice treated only with a single adoptive transfer of splenocytes (Fig. 6G).

### Example 5.

### PPI treatment on the effector function of prostate infiltrating tumor-specific T cells

After minor histocompatibility mismatched hematopoietic stem cell transplantation (HSCT), donor lymphocyte infusion (DLI) and dendritic cell (DC) vaccination, tumor-specific CD8+ T cells mostly of donor origin rapidly infiltrate the prostate of transgenic adenocarcinoma of the mouse prostate (TRAMP) mice affected by autochthonous prostate cancer and, together with minor H specific T cells eradicate the spontaneous tumor (Hess Michelini et al., Cancer Res; 70(9) 2010).

TRAMP mice were subjected to nonmyeloablative total body irradiation (TBI; 600 Rad) and a day later transplanted with 1 × 10⁷ bone marrow cells derived from congenic female donors (fHSCT). Two weeks after the HSCT, mice received a DLI of 6 x 10⁷ splenocytes derived from congenic females previously sensitized against male antigen. A day after the DLI, mice were vaccinated with DC pulsed with the immunodominant Tag epitope IV. After 3 days, mice were treated with either PBS or PPI and killed after additional 24 h (Figure 8A). Tumor cell suspensions were analyzed *ex vivo* by flow cytometry for IFN-_{Y} production following Tag-IV stimulation. As shown in Figure 8B and quantified in Figure 8C, the population of TIL specifically reacting against the tumor antigen markedly increased after PPI treatment.

### Example 6.

### Activation and accumulation of myeloid-derived suppressor cells (MDSCs) in vitro.

These studies were carried out taking advantage of an in vitro model of MDSC conversion set up in our Lab (Valenti R, et al.; Cancer Res. 2006 Sep 15;66(18):9290-8), based the induction of a MDSC-like phenotype in normal monocytes incubated with tumor exosomes, which are nanovesicles released by tumor cells (Valenti R, et al.; Cancer Res. 2007 Apr 1;67(7):2912-5 and lero M, et al.; Cell Death Differ. 2008 Jan;15(1):80-8). Conversion is demonstrated by the acquired ability of secreting a broad panel of immunosuppressive and pro-tumorigenic cytokines/chemokines, highly reminiscent of the functional profile of MDSC isolated from melanoma patients's peripheral blood (Figure 9).

### Results:

Using this approach we demonstrated that pre-treatment with PPI makes normal monocytes less susceptible to exosome-mediated MDSC *in vitro* conversion, as suggested by the significantly reduced cytokine/chemokine secretion (Figure 10).

To seek for the molecular mechanism underlying this effect, we tested whether PPI could inhibit specific signaling pathways in monocytes. As depicted in Figure 11, PPIs inhibit STAT6, STAT3 and STAT1 phosphorylation in response to IL13, IL6, IFNa respectively. However, PPI pretreatment did not affect monocyte viability or phagocytotic activity, or the release of cytokines/chemokines such as IL-8, GRO and MCD.(not shown), suggesting that PPI pre-treatment did not totally abrogate monocyte activity but rather counterattack their skewing toward a MDSC-like phenotype.

### Example 7.

### Activation and accumulation of myeloid-derived suppressor cells (MDSCs) in vivo.

We also tested the effect of PPI in advanced melanoma patients receiving high dose esomeprazole (120mg/day for 3 days) in the context of a clinical trial testing the ability of PPI pretreatment to sensitized patients to chemotherapy (CISPLATIN).

### Results:

We observed that PPI treatment for 3 days significantly and rapidly decreases the frequency of MDSC in peripheral blood. This effect was selective, as no change in total CD14⁺, CD3⁺, CD4⁺, CD19⁺ cells or granulocytes was detected in peripheral blood. In addition circulating monocytes displayed reduced ex-*vivo* secretion of immunosuppressive/pro-tumorigenic cytokines such as TGFβ and VEGF-A when obtained after 3 days of high dose PPI treatment (1809±900 vs 1502±755 pg/ml for TGFβ, and 120±100 vs 104±58 for VEGF-A).

These cells also show a reduction in STAT3 and STAT6 phosphorylation states (20% and 15% respectively) with respect to cells obtained prior to PPI administration.

Patients receiving PPI + chemotherapy also showed a significant increase of tumor immunity in peripheral blood as compared with patients treated with chemotherapy alone, as detected by IFNγ Elispot with allogenic HLA-matched melanoma cells. This latter evidence implies that PPI can contribute in the in vivo recovery of spontaneous T cell-mediated tumor immunity.

All together these data further extend the beneficial effects on tumor immunity mediated by PPI administration. Indeed, in association with a rescuing effect of acidity-mediated anergy in tumor-specific T cells, PPI can interfere with the *in vitro* and *in vivo* generation of immunosuppressive cells recruited by tumors. These drugs thus counterattack the detrimental effects mediated by tumor cells on host immune responses, by rendering myeloid cells, usually recruited by tumor microenvironment as suppressive pro-tumorigenic cells, resistant and unresponsive.

From the above description and the above-noted examples, the advantage attained by the product described and obtained according to the present invention are apparent.

## Claims

1. A proton pump inhibitor for use as an immunomodulator.

2. The proton pump inhibitor according to claim 1, wherein said proton pump inhibitor is a 2- pyridyl methylsulphinyl benzimidazole proton pump inhibitor.

3. The proton pump inhibitor according to claim 2, wherein said proton pump inhibitor is selected from the group consisting of omeprazole, lansoprazole, pantoprazole, esomeprazole, dexlansoprazole, rabeprazole, tenatoprazole or a mixture thereof.

4. The proton pump inhibitor according to claim 3, wherein said proton pump inhibitor is esomeprazole.

5. The proton pump inhibitor according to anyone of claims 1-4, for use in the combination therapy or prophylaxis of a disease condition, wherein the proton pump inhibitor is administered prior to at least one drug indicated against said condition.

6. The proton pump inhibitor according to claim 5, wherein said condition is a cancerous condition.

7. The proton pump inhibitor according to claim 5, wherein said condition is a tumor.

8. The proton pump inhibitor according to claim 7, for blocking or reducing tumor growth.

9. The proton pump inhibitor according to claim 5, wherein said condition is a chronic infection.

10. The proton pump inhibitor according to claim 9, wherein said chronic infection is associated with a cancerous condition or a tumor.

11. The proton pump inhibitor according to claim 9 or 10, wherein said chronic infection is tuberculosis or AIDS.

12. A vaccine composition comprising an effective amount of at least one proton pump inhibitor and pharmaceutically acceptable excipients.

13. The vaccine composition according to claim 12, wherein said vaccine is a cancer preventive vaccine or a cancer treatment vaccine.

14. The vaccine composition according to anyone of claims 12 or 13, wherein said vaccine is a peptide derivative, a protein or a DNA vaccine.

15. The vaccine composition according to anyone of claims 13 or 14, for use in the treatment or prevention of a cancerous condition or a tumor or in the treatment of a chronic infection.
